# EUROPÄISCHE PATENTSCHRIFT

(11) **EP 0 851 864 B1**
(45) Veröffentlichungstag und Bekanntmachung des Hinweises auf die Patenterteilung: **23.05.2001**
(21) Anmeldenummer: 96931061.4
(22) Anmeldetag: 10.09.1996
(51) Int. Cl.: C07D 317/22, A61K 7/13

(54) **NEUE KUPPLER, VERFAHREN ZU IHRER HERSTELLUNG UND HAARFÄRBEMITTEL**
NOVEL COUPLING AGENTS, PROCESSES FOR THEIR PREPARATION AND HAIR DYES
NOUVEAUX COUPLEURS, PROCEDE DE PRODUCTION CORRESPONDANT ET TEINTURE CAPILLAIRE

(30) Priorität: 15.09.1995 DE 19534213
(43) Veröffentlichungstag der Anmeldung: 08.07.1998
(73) Patentinhaber: Hans Schwarzkopf GmbH & Co. KG, 22763 Hamburg (DE)
(72) Erfinder: AKRAM, Mustafa, D-22457 Hamburg (DE); BAUER, Wolfgang, D-63477 Maintal (DE); BITTNER, Andreas, D-63071 Offenbach (DE); KLEEN, Astrid, D-40627 Düsseldorf (DE)
(74) Vertreter: Foitzik, Joachim Kurt
(86) Internationale Anmeldenummer: EP9603960
(87) Internationale Veröffentlichungsnummer: WO9710237

(56) Entgegenhaltungen:
- WO-A-93/10744
- CHEMICAL ABSTRACTS, vol. 113, no. 21, 1990 Columbus, Ohio, US; abstract no. 191208h, KIRILLOV,I.: "REACTION OF O-LITHIATED DERIVATIVES OF 2-SUBSTITUTED 4-(HYDROXYMETHYL)-1,3-DIOXOLANES WITH NITROAROMATIC COMPOUNDS." Seite 710; XP002020795 & ZH. OBSHCH. KHIM., Bd. 60, Nr. 5, 1990, RUSS., Seiten 1140-1144,

## Beschreibung

Die vorliegende Erfindung betrifft neue substituierte 4-(2,4-Diaminophenoxymethyl)-1,3-dioxolane der allgemeinen Formel (I), Verfahren zu ihrer Herstellung und ihre Verwendung als Haarfärbemittel.

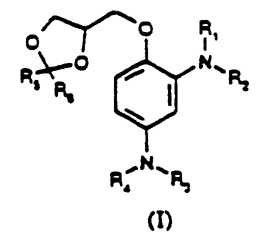

Zum Färben von keratinischen Fasern werden sogenannte Oxidationsfarben eingesetzt, da diese im Färbeergebnis intensive Farben von hoher Farbechtheit liefern. Unter den anwendungstechnischen Randbedingungen (tiefe Färbetemperatur und kurze Färbedauer) ergeben sie intensive Farben mit guten Echtheiten. Dabei werden die Farbstoffe während des Färbevorgangs durch die oxidative Kupplung einer Entwicklerkomponente und einer Kupplerkomponente gebildet. Derartige Kuppler- und Entwicklerkomponenten zur Erzielung verschiedenster Farbtöne, im folgenden Haarfarbstoffzwischenprodukte bezeichnet, sind in der Literatur (K. Venkataraman, The Chemistry of Synthetic Dyes, Vol. V, Academic Press. *1971*; F. Brody und M. S. Burns, J. Soc. Cosmet. Chem. **19**, 361 - 379, *1968*; H. Husemeyer, J. Soc. Cosmet. Chem. **25,** 131 - 138. *1974*) zahlreich beschrieben.

Obwohl die oxidative Kupplung, d. h. die Entwicklung der Färbung, im Prinzip auch durch Luftsauerstoff erfolgen kann. welche aber in aller Regel zu langsam erfolgt und/oder zu ungleiche Färbeergebnisse liefert, werden gewöhnlich chemische Oxidationsmittel eingesetzt. Bevorzugt werden dabei Oxidationsmittel auf der Basis von Wasserstoffperoxid, neben Wasserstoffperoxid selbst auch Additionsverbindungen mit Harnstoff, Melamin oder Alkaliperborat, verwendet.

In der Literatur ist die Verwendung von 1-Alkoxysubstituierten 2,4-Diaminobenzolderivaten als Kupplersubstanzen schon länger bekannt. So offenbart die WO 93/10744 beispielsweise Haarfärbemittel, die als Kupplerkomponente spezielle Alkoxymetaphenylendiamine enthalten. Weiterhin sind in den Druckschriften DE-PS 38 06 237 und DE-OS 32 44 517 substituierte 2,4-Diaminoanisole und in DE-OS 27 37 138 substituierte β-Hydroxy-2,4-diaminophenetole beschrieben. Die dort beschriebenen Färbungen erzielten einen intensiv-blauen Farbton, jedoch mit deutlichem Rotanteil, der insbesondere bei helleren Nuancen sichtbar wird. Für die Erzielung von Naturnuancen, die eine ausreichende Farbtiefe und eine ausreichende Grauabdeckung erreichen sollen, ist der Rotanteil nachteilig. Dringend notwendig war daher die Entwicklung neuer Kuppler, die eine intensive Färbung im klaren Blaubereich ergeben. Überraschenderweise wurde nun gefunden, daß die in der vorliegenden Erfindung beschriebenen Verbindungen der allgemeinen Formel (I) diese Forderung besonders gut erfüllen. Die vorliegende Erfindung betrifft demnach die Verwendung und Verfahren zur Herstellung von Verbindungen der Formel (I) sowie deren Salze mit anorganischen oder organischen Säuren, 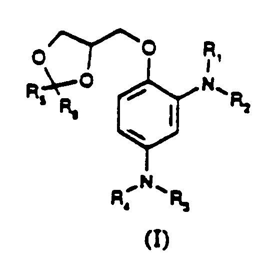 worin R¹, R², R³ und R⁴ unabhängig voneinander ein Wasserstoffatom, eine (C₁-C₄)-Alkylgruppe, eine Hydroxy-(C₂-C₃)-alkylgruppe, eine Alkoxy-(C₂-C₃)-alkylgruppe, eine Amino-(C₂-C₃)-alkylgruppe, eine 2,3-Dihydroxypropylgruppe ist und R⁵ sowie R⁶ unabhängig voneinander Wasserstoff oder eine (C₁-C₄)-Alkylgruppe darstellen können.

Spezielle [(Aryloxy)methyl)] dioxolane sind zwar aus Zh. Obshch. Khim 1990, 60 (5), 1140-4 (referiert bei Chem. Abstr. Vol. 113, Referat 191208h) bekannt. Diese Verbindungen sind aufgrund der Substitutionsmuster am Phenylring jedoch prinzipiell als Kupplerkomponenten für Oxidationsfarbstoffe nicht einsetzbar.

Die erfindungsgemäßen Kupplersubstanzen werden hergestellt, indem 2,4-Dinitrohalogenbenzole der Formel (II), worin X = Fluor, Chlor, Brom oder lod ist, mit 4-Hydroxymethyl-1,3-dioxolanen der Formel (III), worin R⁵ und R⁶ die oben angegebene Bedeutung haben, in alkalischem Reaktionsmedium, gegebenenfalls unter Zusatz von Phasentransferkatalysatoren, zu 4-(2,4-Dinitrophenoxymethyl)-1,3-dioxolanen der Formel (IV) umgesetzt und diese zu den erfindungsgemäßen Verbindungen der Formel (I) reduziert.

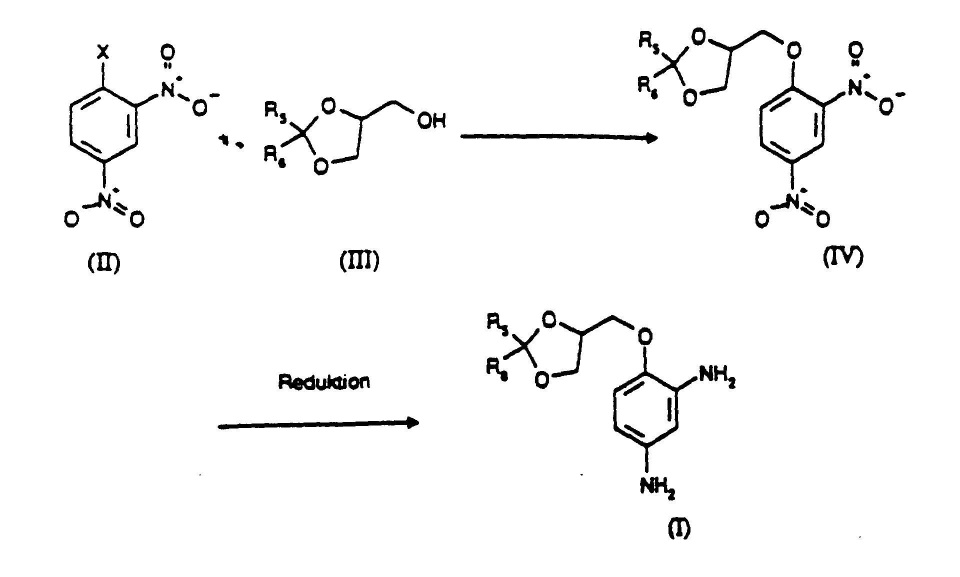

Die Verbindungen der Formel (III) sind bereits bekannt und können durch Umsetzung von Glycerin mit Carbonylverbindungen hergestellt werden (siehe beispielsweise E. Fischer, Ber. Dtsch. Chem. Ges. **28,** 1169 (*1895*); H. S. Hill, E. C. Hill und H. Hibbert, J. Am. Chem. Soc. **50,** 2246 (1928); M. S. Newman und M. Renoll, ibid **67,** 1621 (*1945*)).

Nach einem weiteren Verfahren können die erfindungsgemäßen Kuppler der Formel (I) dadurch erhalten werden, daß man 4-Halogen-3-nitraniline bzw. 2-Halogen-5-nitraniline mit 4-Hydroxymethyl-1,3-dioxolanen zunächst zu Verbindungen der Formel (Va) 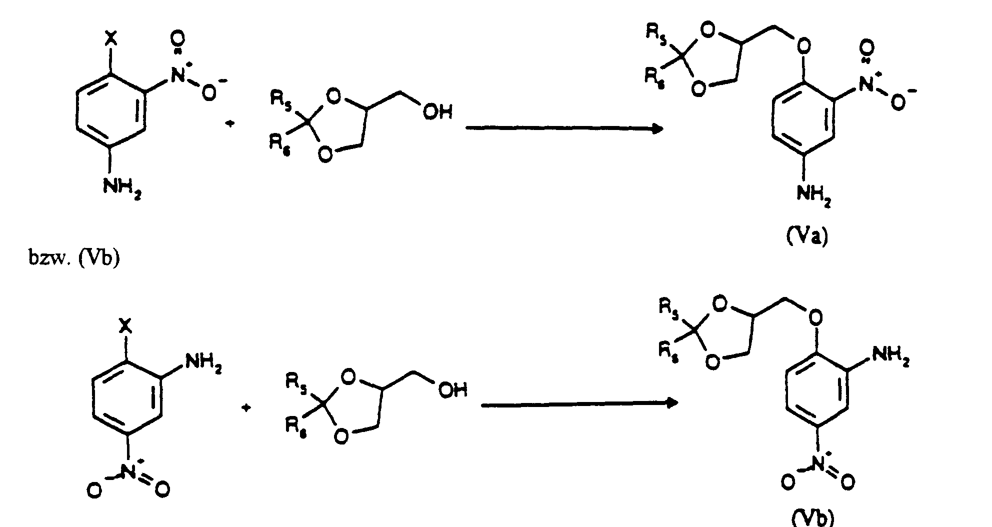 umsetzt.

Diese werden dann anschließend in einem inerten Lösungsmittel mit Chlorameisensäure-2-chlorethylester oder Chlorameisensäure-3-chlorpropylester in Verbindungen der Formel (VIa) 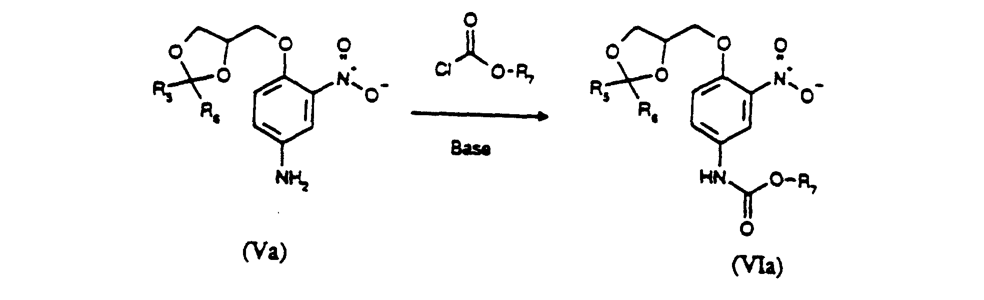 oder (VIb) 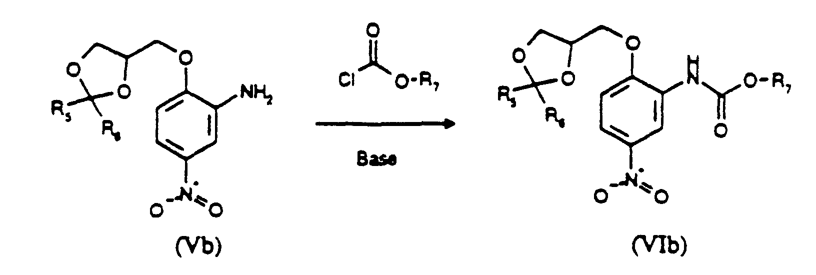 überführt, worin R⁷ = CH₂CH₂Cl oder CH₂CH₂CH₂Cl bedeutet.

Die Zwischenprodukte der Formel (VIa) bzw. (VIb) werden darauf mit starken Basen, z. B. Natrium- oder Kaliumhydroxid, zu Verbindungen der allgemeinen Formel (VIIa) 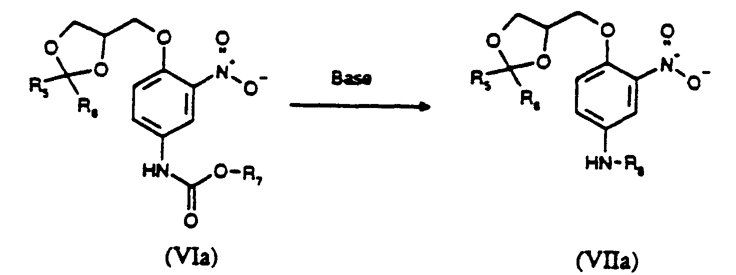 bzw. (VIIb) 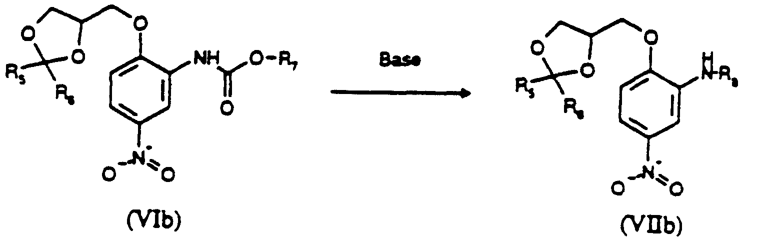 umgesetzt, worin R⁸ = CH₂CH₂OH bzw. CH₂CH₂CH₂OH bedeutet, die mit bekannten Alkylierungs- oder Oxalkylierungsmitteln zu Zwischenprodukten der allgemeinen Formel (VIIIa) oder (VIIIb) 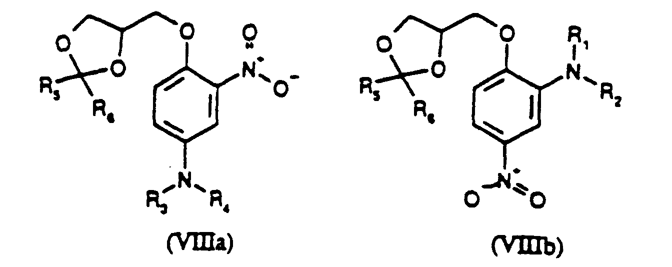 reagieren, worin R¹ bis R⁴ die bereits angegebenen Bedeutungen haben.

Nach Reduktion und gegebenenfalls weiterer Alkylierung oder Oxalkylierung werden dann die erfindungsgemäßen Kuppler der Formel (I) erhalten und diese gegebenenfalls mit einer anorganischen oder organischen Säure in ihr Salz überführt.

Für die praktische Anwendung als Haarfärbemittel werden die Haarfarbstoffzwischenprodukte in eine geeignete kosmetische Grundlage eingearbeitet, die je nach Art des gewünschten Endproduktes eine Lösung, eine Creme, ein Schaum oder ein Gel sein kann. Diese Zubereitung wird unmittelbar vor der Applikation am Haar mit dem Oxidationsmittel vermischt.

Zur Bildung von blauen Farbtönen werden überwiegend 1,3-Diaminobenzol-Derivate als Kuppler und 1,4-Diaminobenzol-Derivate als Entwicklersubstanzen eingesetzt. Es wurde nun gefunden, daß 4-(2,4-Diaminophenoxymethyl)-1,3-dioxolane der allgemeinen Formel (I) in Kombination mit an sich bekannten Entwicklersubstanzen blaue bis blauschwarze Färbungen von überraschend hoher Farbstärke sowie hervorragenden Echtheitseigenschaften ergeben. Diese Färbungen sind mittels eines Oxidationsmittels oder durch den Zusatz eines Katalysators in die Färbemasse und anschließende Luftoxidation (d. h. durch Weglassen der sonst üblichen wäßrigen Oxidationsmittel auf Basis von Wasserstoffperoxid) zu erhalten.

Für die erfindungsgemäßen Haarfärbemittel können an sich bekannte Kuppler- und Entwickler-Kombinationen Verwendung finden. Bevorzugte Kuppler und Entwickler sind folgende Kombinationen:
1. p-Toluylendiamin, Resorcin, m-Aminoanilin, 4-Chlorresorcin
2. p-Toluylendiamin, 2-Methylresorcin, 4-Chlorresorcin, 2-Amino-3-hydroxypyridin
3. p-Toluylendiamin, Resorcin, m-Aminoanilin, p-Aminoanilin, 2-Hydroxy-4-aminotoluol
4. 3-Methyl-4-aminoanilin, m-Aminoanilin, 2-Hydroxy-4-aminotoluol, 2-Amino-3-hydroxypyridin
5. 3-Methyl-4-aminoanilin, 2-Methylresorcin, m-Aminoanilin, p-Toluylendiamin, 2-Hydroxy-4-aminotoluol, 2-Amino-3-hydroxypyridin

Eine spezielle bevorzugte Ausführungsform der Erfindung ist daher ein Haarfärbemittel auf der Basis von Oxidationsfarben, gekennzeichnet durch
1. die Verwendung von neuen substituierten 4-(2,4-Diaminophenoxymethyl)-1,3-dioxolan-Derivaten,
2. das Vorliegen eines einkomponentigen Haarfärbesystems,
3. den Verzicht auf eine Vorbehandlung des Haares mit Katalysatoren,
4. den Zusatz eines Katalysators zur Färbemasse,
5. den Verzicht auf Wasserstoffperoxid als Oxidationsmittel,
6. Übergangsmetallsalze und Übergangsmetallkomplexe, insbesondere Kupfer(II)-chlorid, -sulfat, -acetat, allein oder als Addukt mit Ammoniak, 1,2-Ethylendiamin, Phenanthrolin, Triphenylphosphin, 1,2-Diphenylphosphinoethan, 1,3-Diphenylphosphinopropan oder Aminosäuren; z. B. seien ohne einschränken zu wollen genannt: Glycerin, Leucin, Methionin, Alanin, Phenylalanin oder Prolin als Katalysator zwecks Zusatz zur Färbemasse.

Bei der Grundmasse des erfindungsgemäßen Haarfärbemittels handelt es sich je nach Art des gewünschten Endproduktes beispielsweise um ein Gel, eine Creme, einen Schaum oder eine tensidhaltige Lösung. Die Grundmasse besteht aus dem Stand der Technik bekannten und für die Anwendung auf dem Haar geeigneten Bestandteilen in für diese Zwecke üblichen Mengen. Die Bestandteile solcher Grundlagen sind z. B.
1. Netz- und Emulgiermittel
2. Verdickungsmittel
3. Reduktionsmittel
4. Parfümöle
5. haarpflegende Zusätze und
6. Lösungsmittel, z. B. Wasser oder niedere Alkohole.

Das Färben von Haaren mit dem erfindungsgemäßen Haarfärbemittel erfolgt, indem die Komponente, mit Wasser verdünnt oder unverdünnt auf das Haar aufgetragen und verteilt wird. Die erforderliche Menge an Haarfärbemittel ist, wie dem Fachmann bekannt ist, von der Länge des zu färbenden Haares abhängig. Die Anwendungstemperaturen liegen vorzugsweise in einem Bereich von 15 bis 40 °C und die Einwirkungszeit vorzugsweise zwischen 5 und 45 Minuten, insbesondere 10 bis 35 Minuten. Das Haarfärbemittel wird dann durch Ausspülen entfernt, das Haar gegebenenfalls mit einem Shampoo nachgewaschen und nochmals gespült und dann getrocknet.

Das erfindungsgemäße Haarfärbemittel ist in der speziellen bevorzugten Ausführungsform im Vergleich zu bisher bekannten Haarfärbemitteln einfacher in der Anwendung, milder und verträglicher durch die Luftoxidations-Farbentwicklung in Anwesenheit des in der Färbemasse als Zusatz enthaltenen Katalysators, den Verzicht auf Wasserstoffperoxid, ohne daß Einbußen beim Färbeergebnis hingenommen werden müssen.

Die Umsetzung der Verbindung der Formel (Va) oder (Vb) mit einem Chlorameisensäure-2-chlorethylester oder Chlorameisensäure-3-chlorpropylester erfolgt in Anlehnung an die bekannte selektive Hydroxyalkylierung eines Amins mit Chlorameisensäurechloralkylesters mit anschließender basischer Behandlung der Chloralkylcarbamate (vgl. Otto, J. Prakt. Chem. **44**, 15, *(1890);* R. Adams und J. B. Segur, J. Am. Chem. Soc. **45,** 785, (*1923)*. *J.* S. Pierce und R. Adams, J. Am. Chem. Soc. **45,** 790, (*1923*) beschreiben ausführlich die Umsetzung des Chlorameisensäurechloralkylesters mit primären aromatischen Aminen. Zur Bildung der Verbindungen der allgemeinen Formel (VIa) oder (VIb) wird die Verbindung der Formel (Va) oder (Vb) in einem inerten organischen Lösungsmittel, z. B. Dioxan, C₁- bis C₄-Alkohole, Dimethylformamid, Tetrahydrofuran, Toluol, Chlorbenzol, Methylethylketon, 1,2-Dimethoxyethan, Methyl-*tert*.- butylether oder Diethylenglykoldimethylether, vorgelegt und auf eine Temperatur zwischen Raumtemperatur und Rückflußtemperatur, vorzugsweise zwischen 30 °C und Rückflußtemperatur, erhitzt. Anschließend wird einer der genannten Chlorameisensäurechloralkylester in äquimolarer Menge oder in geringem Überschuß zudosiert. Gegebenenfalls können die Lösungsmittel mit Wasser kombiniert werden. Dabei kann ein säurebindendes Mittel entweder mit vorgelegt oder parallel zum bereits genannten Chlorameisensäurechloralkylester zugefügt werden. Als säurebindende Mittel kommen Basen wie Alkalihydroxide, -hydrogencarbonate, -carbonate, Erdalkalioxide, -hydroxide, -hydrogencarbonate und -carbonate sowie tertiäre organische Amine in Betracht. Die Reaktionsdauer beträgt zwischen 1 und 12 Stunden.

Nach vollständiger Umsetzung werden die Carbamate isoliert, indem a) Wasser oder Eis oder ein Gemisch von Eis und Wasser unter Rühren in den Ansatz gegeben wird oder b) die anorganischen Salze abfiltriert werden und das Lösungsmittel teilweise oder ganz abdestilliert wird, gegebenenfalls unter Kühlung, z. B. durch Zugabe von Eis, wodurch die gebildeten Carbamate der Formel (VIa) oder (VIb) in fester Form nahezu quantitativ ausfällen.

Durch Behandeln mit starken Basen - hier kommen Alkali- oder Erdalkalihydroxide in Betracht, vorzugsweise wird 10 bis 50 %ige Natron- oder Kalilauge eingesetzt - werden die Carbamate der allgemeinen Formel (VIa) oder (VIb) in die Hydroxyalkylverbindungen der Formel (VIIa) oder (VIIb) überführt.

Dabei sind zwei Verfahrensweisen zweckmäßig:
a) Das Carbamat der Formel (VIa) oder (VIb) wird in Wasser oder einem organischen Lösungsmittel, z. B. einem C₁- bis C₄-Alkohol, einem wassermischbaren Ether oder Mischungen davon vorgelegt, bei Raumtemperatur wird dann ungefähr die berechnete Menge Lauge, das sind 4 Mol Lauge pro Mol Carbamat, zudosiert, und es wird bis zur vollständigen Umsetzung nachgerührt, wobei ggf. bis zum Rückfluß erhitzt oder Lauge nachgesetzt werden kann.
b) Man legt die Lauge, die mit den genannten Lösungsmitteln verdünnt sein kann, vor, dosiert das Carbamat mit der allgemeinen Formel (VIa) oder (VIb) in reiner Form oder gelöst in einem der genannten organischen Lösungsmittel bei einer Temperatur zwischen Raumtemperatur und ca. 70 °C zu und rührt dann bis zur vollständigen Umsetzung nach.

Bei beiden Varianten kann die Reaktionslösung, die einen pH-Wert von ca. 12 bis 14 aufweist, zur Aufarbeitung durch Zusatz einer organischen oder anorganischen Säure auf einen pH-Wert von etwa 5 bis 10 abgestumpft werden. Anschließend trennt man die Salze ab, setzt gegebenenfalls Wasser zu und isoliert das Produkt der allgemeinen Formel (VIIa) oder (VIIb) nach Entfernen des organischen Lösungsmittels.

Bei den beiden vorstehenden Verfahrensweisen wird durch Zusatz von etwa 25 bis 30 Gew.-% eines der vorstehend genannten organischen Lösungsmittels in den wäßrigen Reaktionsansatz die Reaktionsdauer deutlich verkürzt, wobei die anorganischen Salze noch im Reaktionsmedium gelöst bleiben. Für die Umsetzung werden etwa 1 bis 12 Stunden benötigt.

Die Herstellung der Verbindungen der allgemeinen Formel (I) kann durch Reduktion der Verbindungen der allgemeinen Formel (Va) oder (Vb), gegebenenfalls nach Alkylierung oder Oxalkylierung mit unedlen Metallen oder durch katalytische Reduktion erfolgen.

Bei der katalytischen Reduktion werden übliche Katalysatoren, z.B. Raney-Nickel, Palladium auf Aktivkohle oder Platin auf Aktivkohle, eingesetzt. Die Reaktionstemperatur liegt zwischen Raumtemperatur und 120 °C, vorzugsweise zwischen 35 und 100 °C, der Druck liegt zwischen Normaldruck und 100 bar, vorzugsweise zwischen 20 und 70 bar. Als Lösungsmittel finden übliche Lösungsmittel wie Wasser, Toluol, Eisessig, niedere Alkohole oder Ether Verwendung. Nach erfolgter Reduktion und Abtrennung des Katalysators kann das Produkt der allgemeinen Formel (I), gegebenenfalls nach Alkylierung oder Oxalkylierung, durch Abziehen des Lösungsmittels unter einem Schutzgas in freier Form isoliert werden. Als Alkylierungsmittel haben sich die bekannten Verbindungen Dimethyl- und Diethylsulfat und als Oxalkylierungsmittel die bekannten Verbindungen Ethylenoxid und Propylenoxid bewährt. Das Produkt nach der allgemeinen Formel (I) wird vorzugsweise unter einem Schutzgas durch Zugabe einer ungefähr äquivalenten Menge einer Säure in ein Salz überführt, das entweder direkt ausfällt oder nach Abzug des Lösungsmittels erhalten wird. Als anorganische Säuren sind z.B. Salzsäure, Schwefelsäure, Phosphorsäure und als organische Säuren Essigsäure, Propionsäure, Milchsäure oder Citronensäure zur Salzbildung geeignet.Die folgenden Beispiele sollen die Erfindung beschreiben und erläutern, ohne sie darauf beschränken zu wollen.

### A. Herstellung der Katalysatoren

### Beispiel A.1

### Darstellung von Kupfer(II)-glycinat

In 100 mL heißem Methanol werden 1.8 g (9.8 mMol) Kupfer(II)-acetat gelöst und eine Lösung von 1.5 g (20 mMol) Glycin in 50 mL Methanol unter Rühren zugegeben. Die Lösung trübt sich durch ausgefallenes Produkt. Man rührt noch zwei Stunden bei fallender Temperatur, filtriert das ausgefallene Produkt ab und trocknet es.
Ausbeute: 2.49 g (96 % d.Th.)
Schmp.: > 210 °C

### Beispiel A.2

### Darstellung von Kupfer(II)-1,2-diaminoethan-chlorid

In 300 mL heißem Methanol werden 26,9 g (160 mMol) Kupfer(II)-chlorid-Dihydrat gelöst und eine Lösung von 9.6 g (160 mMol) 1,2-Diaminoethan in 100 mL Methanol unter Rühren zugegeben. Die Lösung trübt sich durch ausgefallenes Produkt. Man rührt noch eine Stunde unter Rückfluß nach. Die Mischung läßt man unter Rühren abkühlen, filtriert das ausgefallene Produkt ab und trocknet es.
Ausbeute: 12.5 g (40 % d.Th.)
Schmp.: > 210 °C

### B. Herstellung neuer Kuppler

Alle hergestellten Verbindungen sind durch IR- bzw. IR- (KBr-Preßling) und ¹H-NMR-Spektren (in D₆-DMSO) charakterisiert worden. Bei den IR-Spektren sind nur die sehr starken und starken Banden aufgeführt. Bei den Angaben zu den ¹H-NMR-Spektren bedeuten s Singulett, d Dublett. dd Dublett vom Dublett, t Triplett, m Multiplett, ³J bzw. ⁴J die Kopplungen über drei bzw. vier Bindungen, sowie H³, H⁵ und H⁶ die Wasserstoffatome in Position 3,5 bzw. 6 des Benzolrings.

### Beispiel B.1

### Darstellung von 4-(2,4-Dinitrophenoxymethyl)-2,2-dimethyl-1,3-dioxolan

### Stufe a)

### Darstellung von 4-(2,4-Dinitrophenoxymethyl)-2,2-dimethyl-1,3-dioxolan

In einer Mischung aus 650 mL 1,2-Dimethoxyethan, 528.6 g (4 Mol) Isopropylidenglycerin und 7 g Methyl-tri(C₆-C₈)alkylammoniumchlorid werden 204 g (1 Mol) 2,4-Dinitrochlorbenzol gelöst. Unter Rühren und Kühlen tropft man 123 g (1.1 mol) 50 %ige Kalilauge binnen einer Stunde so zu, daß die Innentemperatur nicht über 35 °C steigt. Die Mischung wird über Nacht bei Raumtemperatur gerührt und dann in 3250 mL Wasser eingerührt. Das ausgefallene Produkt wird abgesaugt, zweimal mit ca. 250 mL Wasser gewaschen und bei 40 °C im Vakuum getrocknet.

| | |
|---|---|
| Ausbeute | 246.7 g (86.8 % d.Th.) |
| Schmelzpunkt | 93 - 95 °C |
| IR | 3415 cm⁻¹ (νOH), 3122 cm⁻¹ (ν CH_{Ar}), 2989, 2939 cm⁻¹ (ν CH), 1612 cm⁻¹ (vC=C), 1537 cm⁻¹ (νₐₛ NO₂), 1345 cm⁻¹ (νₛNO₂). |
| ¹H-NMR | 8.76 ppm (H³, d, ⁴J_{H,H}= 2,81 Hz); 8.49 ppm (H⁵, dd, ³J_{H,H}= 9.33 Hz, ⁴J_{H,H}= 2.81 Hz); 7.63 ppm (H⁶, d, ³J_{H,H}= 9.39 Hz); 4.48 - 4.34 ppm (3H, m, φOCH₂CH, φOCH₂CH); 4.10 ppm (1H, syn-CH₂OC(CH₃)₂, m); 3.82 ppm (1 H, anti-CH₂OC(CH₃)₂, m); 1.325 ppm (3H, s, syn-C(CH₃)₂); 1.308 ppm (3H, s, anti-C(CH₃)₂). |

### Stufe b)

### Darstellung von 4-(2,4-Diaminophenoxymethyl)-2,2-dimethyl-1,3-dioxolan

In einem 0,7 L Autoklaven werden 215 mL Methanol vorgelegt, 85.3 g (0.3 Mol) 4-(2.4-Dinitrophenoxymethyl)-2,2-dimethyl-1,3-dioxolan (Stufe a) gelöst und 0.6 g Palladium auf Aktivkohle 10 % (Degussa) zugegeben. Nach Verschließen und Inertisieren mit Stickstoff wird bei einem Druck von 4 bar und einer Temperatur von 35 - 40 °C hydriert, bis kein Wasserstoff mehr aufgenommen wird. Zu der warmen Lösung gibt man unter Stickstoff 1.3 g Aktivkohle und filtriert den Katalysator ab. Die Lösung wird unter Eiskühlung bei 5 °C mit 42 g 70 %iger Schwefelsäure tropfenweise versetzt. Das ausgefallene Produkt wird abgesaugt, mit Methanol gewaschen und getrocknet.

| | |
|---|---|
| Ausbeute | 68.9 (68.3 % d. Th.) |
| Schmp. | > 250 °C |
| IR | 3415 cm⁻¹ (ν OH), 3122 cm⁻¹ (ν CH_{Ar}), 2989, 2939 cm⁻¹ (v CH_{Alkyl}), 1612 cm⁻¹ (ν C=C). |
| ¹H-NMR | 8.76 ppm (H³, d, ⁴J_{H,H}= 2.81 Hz); 8.49 ppm (H⁵, dd, ³J_{H,H}= 9.33 Hz, ⁴J_{H,H}= 2.81 Hz); 7.63 ppm (H⁶, d, ³J_{H,H}= 9.39 Hz); 4.48 - 4.34 ppm (3H, m, φOCH₂CH, φOCH₂CH); 4.10 ppm (1H, m, syn-CH₂OC(CH₃)₂); 3.82 ppm (1H, m, anti-CH₂OC(CH₃)₂); 1.325 ppm (3H, s, syn-C(CH₃)₂); 1.308 ppm (3H, s, anti-C(CH₃)₂). |

### C. Färbebeispiele

Die erfindungsgemäßen Haarfärbemittel sind wäßrige Mittel. Darunter werden sämtliche Mittel verstanden, die in irgendeiner Weise Wasser enthalten, wie z.B. Cremes, Emulsionen, Gele oder auch einfache Lösungen. Die Zusammensetzungen der Haarfärbemittel stellt eine Mischung der Farbstoffkomponenten mit den für solche kosmetischen Zubereitungen üblichen Zusätzen dar. Übliche Zusätze in Lösungen, Cremes, Emulsionen oder Gelen sind z.B. Lösungsmittel wie Wasser, niedere aliphatische Alkohole, beispielsweise Ethanol, Propanol und Isopropanol, oder Glykole wie Glycerin und Glykolether wie Propylenglykol, weiterhin Netzmittel oder Emulgatoren aus den Klassen der anionischen, kationischen, amphoteren oder nichtionogenen oberflächenaktiven Substanzen wie Fettalkoholsulfate, Alkylsulfonate, Alkylbenzolsulfonate, Alkyltrimethylammoniumsalze, Alkylbetaine, oxethylierte Fettalkohole, oxethylierte Nonylfluorbenzole, Fettsäurealkanolamide, oxethylierte Fettsäureester, ferner Verdicker wie höhere Fettalkohole, Stärke, Cellulosederivate, Vaseline, Paraffinöl und Fettsäuren. Die erwähnten Bestandteile werden in den für solche Zwecke üblichen Mengen verwendet, zum Beispiel die Netzmittel und Emulgatoren in Konzentrationen von etwas 0,5 bis 30 Gew.-%, während die Verdicker in einer Menge von etwa 0,1 bis 25 Gew.-% in den Zubereitungen enthalten sein können.

Je nach Zusammensetzung können die erfindungsgemäßen Haarfärbemittel schwach sauer, neutral oder alkalisch reagieren. Insbesondere weisen sie einen pH-Wert im alkalischen Bereich zwischen 7,5 und 11,5 auf, wobei die Einstellung vorzugsweise mit Ammoniak erfolgt. Es können aber auch organische Amine, z.B. Monoethanolamin und Triethanolamin, oder auch anorganische Basen wie Natriumhydroxid und Kaliumhydroxid, Verwendung finden.

Bei Verfahren zur oxidativen Färbung von Haaren werden die Haarfärbemittel dieser Erfindung, welche eine Kombination von in der Haarfärbung bekannten Entwicklersubtanzen mit mindestens einer Verbindung der allgemeinen Formel (I) als Kupplersubstanz, sowie gegebenenfalls zusätzlich bekannte Kupplersubstanzen und direktaufziehende Farbstoffe und gegebenenfalls die bereits erwähnten Katalysatoren enthalten, oder die Haarfärbemittel gemäß den Ansprüchen 8 bis 10, denen vor oder während der Anwendung keine Oxydationsmittel zugefügt werden, auf das Haar aufgetragen. Wahlweise können die Färbemittel gemäß den Ansprüchen 4 bis 7 vor der Applikation auch mit Oxidationsmittel vermischt werden. Als Oxidationsmittel zur Entwicklung der Haarfärbung kommen hauptsächlich Wasserstoffperoxid (beispielsweise als 6 %ige wäßrige Lösung) und dessen Additionsverbindungen an Harnstoff, Melamin oder Natriumborat sowie Gemische aus derartigen Wasserstoffperoxidanlagerungsverbindungen mit Kaliumperoxodisulfat in Betracht. Die Anwendungstemperaturen bewegen sich dabei im Bereich von 15 bis 40 °C. Nach einer Einwirkungsdauer von ca. 10 - 30 Minuten wird das Haarfärbemittel vom zu färbenden Haar durch Spülen entfernt. Hiernach kann das Haar mit einem milden Shampoo nachgewaschen und getrocknet werden.
Die nachfolgenden Beispiele sollen den Erfindungsgegenstand näher erläutern, ohne ihn jedoch hierauf zu beschränken.

### Beispiel 1

Haarfärbemittel in Cremeform
2.20 g p-Toluylendiamin-Sulfat
3.36 g 4-(2,4-Diaminophenoxymethyl)-2,2-dimethyl-1,3-dioxolan-Sulfat
1.20 g Ölsäure
0.50 g Natriumdithionit
6.20 g Laurylalkoholdiglykolethersulfat, Natriumsalz (28 %ige Lsg.)
18.0 g Cetyl-Stearylalkohol
7.50 g Ammoniak, 25%
Wasser auf 100 g

50 g des vorstehend genannten Haarfärbemittels werden kurz vor Gebrauch im Verhältnis 1:1m/m mit 50 g H₂O₂-Lösung (6 %ig) gemischt und auf 100 %ig ergrautem Haar mittels eines Pinsels aufgetragen. Nach einer Einwirkzeit von 30 Minuten bei Raumtemperatur wird das Färbemittel abgespült und das Haar getrocknet. Das Haar hat einen gleichmäßigen tiefblauen Farbton erhalten.

### Beispiel 2

Haarfärbemittel in Gelform
1.81 g p-Phenylendiamin-Hydrochlorid
3.36 g 4-(2,4-Diaminophenoxymethyl)-2,2-dimethyl-1,3-dioxolan-Sulfat
12.0 g Ölsäure
12.0 g Isopropanol
5.00 g Nonoxynol-4
10.0 g Ammoniak, 25 %
0.5 g Natriumsulfit, wasserfrei
Wasser auf 100 g

50 g des vorstehend genannten Färbemittels werden kurz vor dem Gebrauch mit 50 g Wasserstoffperoxid-Lösung, (6 %ig) gemischt. Man läßt das Gemisch 30 Minuten bei Raumtemperaturen auf 100 %ig ergrautes Haar einwirken. Danach wird die Farbmasse ausgespült, nachshampooniert und das Haar getrocknet. Das Haar ist in einem blauschwarzen Farbton eingefärbt.

### Beispiel 3

Haarfärbemittel in Cremeform
1.09 g p-Aminophenol
3.36 g 4-(2,4-Diaminophenoxymethyl)-2,2-dimethyl-1,3-dioxolan-Sulfat
2.50 g Laurylsulfat Natriumsulfat (70 %ige Paste)
1.00 g Ölsäure
0.60 g Natriumsulfit, wasserfrei
12.0 g Cetyl-Stearylalkohol
6.00 g Myristylalkohol
1.00 g Propylenglykol
10.0 g Ammoniak, 25%
Wasser auf 100 g

60 g des vorstehend genannten Färbemittels werden kurz vor Gebrauch mit 60 g Wasserstoffperoxid-Lösung, (6 %ig) gemischt. Man läßt das Gemisch 30 Minuten bei Raumtemperatur auf 100% ergrautem Haar einwirken. Anschließend wird die Färbemasse ausgespült, das Haar nachshampooniert und getrocknet. Das Haar hat einen mittelrotbraunen Farbton.

### Beispiel 4

Haarfärbemittel in Gelform
2.20 g p-Toluylendiamin Sulfat
3.36 g 4-(2,4-Diaminophenoxymethyl)-2,2-dimethyl-1,3-dioxolan-Sulfat
0.80 g 4-(3-Hydroxypropylamino)-3-nitrophenol
2.00 g Ölsäure
0.10 g Polyacrylsäure
0.50 g Natriumsulfit, wasserfrei
4.0 g Laurylalkoholdoglykolethersulfat, Natriumsalz (28 %ige Lösung)
8.0 g Ammoniak, 25%
Wasser auf 100 g

50 g des vorstehend genannten Färbemittels werden kurz vor Gebrauch mit 50 g Wasserstoffperoxid-Lösung, (6 %ig) gemischt. Man läßt das Gemisch 30 Minuten bei Raumtemperatur auf 100% ergrautem Haar einwirken. Anschließend wird die Färbemasse ausgespült, das Haar nachshampooniert und getrocknet. Das Haar hat einen rotvioletten Farbton erhalten.

### Beispiel 5

Haarfärbemittel in Gelform
1.23 g 4-Amino-3-methylphenol
3.36 g 4-(2,4-Diaminophenoxymethyl)-2,2-dimethyl-1,3-dioxolan-Sulfat
1.00 g 4-Amino-2-nitro-N-(2-hydroxyethyl)-anilin (HC Red 3)
14.0 g Ölsäure
10.0 g iso-Propanol
2.0 g PEG-3-Cocamine
0.5 g Ascorbinsäure
8.0 g Ammoniak, 25%
Wasser auf 100 g

50 g des vorstehend genannten Färbemittels werden kurz vor Gebrauch mit 50 g Wasserstoffperoxid-Lösung, (6 %ig) gemischt. Man läßt das Gemisch 30 Minuten bei Raumtemperatur auf 100% ergrautem Haar einwirken. Anschließend wird die Färbemasse ausgespült, das Haar nachshampooniert und getrocknet. Das Haar hat einen intensiven violettroten Farbton erhalten.

### Beispiel 6 (Vergleich zu Beispiel 1: Oxidation mit Luft und Katalysator)

Haarfärbemittel in Cremeform
2.20 g p-Toluylendiamin-Sulfat
3.36 g 4-(2,4-Diaminophenoxymethyl)-2,2-dimethyl-1,3-dioxolan-Sulfat
1.20 g Ölsäure
0.50 g Natriumdithionit
6.20 g Laurylalkoholdiglykolethersulfat, Natriumsalz (28 %ige Lsg.)
18.0 g Cetyl-Stearylalkohol
0.3 g Kupfer(II)-phenanthrolin-chlorid
7.50 g Ammoniak, 25%
Wasser auf 100 g

50 g des vorstehend genannten Haarfärbemittels werden kurz vor Gebrauch im Verhältnis 1:1 m/m mit 50 g Wasser gemischt und auf 100 %ig ergrautem Haar mittels eines Pinsels aufgetragen. Nach einer Einwirkzeit von 10 Minuten bei Raumtemperatur wird das Färbemittel abgespült und das Haar getrocknet. Das Haar hat einen gleichmäßigen blausilbergrauen Farbton erhalten. Die Tiefe der erhaltenen Farbe baut sich entsprechend der Einwirkzeit auf.

### Beispiel 7 (Vergleich zu Beispiel 1: Oxidation mit Luft ohne Katalysator)

Haarfärbemittel in Cremeform
2.20 g p-Toluylendiamin-Sulfat
3.36 g 4-(2,4-Diaminophenoxymethyl)-2,2-dimethyl-1,3-dioxolan-Sulfat
1.20 g Ölsäure
0.50 g Natriumdithionit
6.20 g Laurylalkoholdiglykolethersulfat, Natriumsalz (28 %ige Lsg.)
18.0 g Cetyl-Stearylalkohol
7.50 g Ammoniak, 25%
Wasser auf 100 g

50 g des vorstehend genannten Haarfärbemittels werden kurz vor Gebrauch im Verhältnis 1:1 m/m mit 50 g Wasser gemischt und auf 100 %ig ergrautem Haar mittels eines Pinsels aufgetragen. Nach einer Einwirkzeit von 30 Minuten bei Raumtemperatur wird das Färbemittel abgespült und das Haar getrocknet. Das Haar hat unter diesen Bedingungen keine Färbung angenommen.

### Beispiel 8 (Vergleich zu Beispiel 1: Oxidation mit H₂O₂ und Katalysator)

Haarfärbemittel in Cremeform
2.20 g p-Toluylendiamin-Sulfat
3.36 g 4-(2,4-Diaminophenoxymethyl)-2,2-dimethyl-1,3-dioxolan-Sulfat
1.20 g Ölsäure
0.50 g Natriumdithionit
6.20 g Laurylalkoholdiglykolethersulfat, Natriumsalz (28 %ige Lsg.)
18.0 g Cetyl-Stearylalkohol
0.3 g Kupfer(II)-phenanthrolin-chlorid
7.50 g Ammoniak, 25%
Wasser auf 100 g

50 g des vorstehend genannten Haarfärbemittels werden kurz vor Gebrauch im Verhältnis 1:1 m/m mit 50 g H₂O₂-Lösung (6 %ig) gemischt und auf 100 %ig ergrautem Haar mittels eines Pinsels aufgetragen. Nach einer Einwirkzeit von 30 Minuten bei Raumtemperatur wird das Färbemittel abgespült und das Haar getrocknet. Das Haar hat einen gleichmäßigen tiefblauen Farbton erhalten.

Der folgenden Tabelle sind weitere erfindungsgemäße Kuppler der allgemeinen Formel (I) 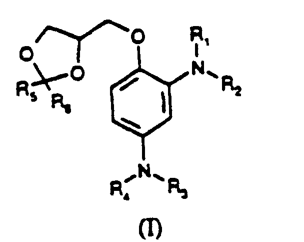 zu entnehmen, wobei in den Spalten 2 bis 5 die Substituenten R¹, R³, R⁵ und R⁶ (R² und R⁴ ist in den aufgeführten Fällen immer Wasserstoff) und in Spalte 6 der analog Beispiel 1 mit p-Toluylendiamin erhaltene Farbton aufgeführt ist.

**Tabelle 1:**

| Ausfärbungen weiterer Verbindungen analog Beispiel 1 auf Keratinfasern | | | | | |
|---|---|---|---|---|---|
| Beispiel | R¹ | R³ | R⁵ | R⁶ | Farbton |
| 1a | H | H | H | H | blauschwarz |
| 1b | H | H | CH₃ | H | blauschwarz |
| 1c | H | H | C₂H₅ | H | blauschwarz |
| 1d | H | H | CH(CH₃)₂ | H | blauschwarz |
| 1e | H | CH₂CH₂OH | CH₃ | CH₃ | blauschwarz |
| 1f | H | CH₂CH₂CH₂OH | CH₃ | CH₃ | blauschwarz |
| 1g | CH₂CH₂OH | CH₂CH₂OH | CH₃ | CH₃ | blauschwarz |
| 1h | CH₂CH₂CH₂OH | H | CH₃ | CH₃ | blauschwarz |

## Patentansprüche

1. Verbindungen der allgemeinen Formel (I) sowie ihre Salze mit anorganischen und organischen Säuren, 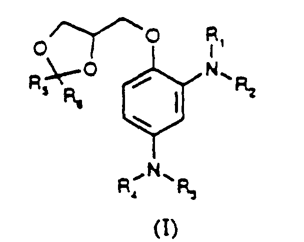 worin R¹, R², R³ und R⁴ unabhängig voneinander ein Wasserstoffatom, eine (C₁-C₄)-Alkylgruppe, eine Hydroxy-(C₂-C₃)-alkylgruppe, eine Alkoxy-(C₂-C₃)-alkylgruppe, eine Amino-(C₂-C₃)-alkylgruppe, eine 2,3-Dihydroxypropylgruppe ist, und R⁵ sowie R6 unabhängig voneinander Wasserstoff oder eine (C₁ - C₄)-Alkylgruppe darstellen können.

2. Verfahren zur Herstellung von Verbindungen der allgemeinen Formel (I), dadurch gekennzeichnet, daß man 2,4-Dinitrohalogenbenzole der Formel (II), worin X = F, Cl, Br oder Iod ist, mit 4-Hydroxymethyl-1,3-dioxolanen der Formel (III), worin R⁵ und R⁶ die bereits genannte Bedeutung besitzt, in alkalischem Reaktionsmedium zu 4-(2,4-Dinitrophenoxymethyl)-1,3-dioxolanen der Formel (IV) umsetzt und diese zu den erfindungsgemäßen Kupplersubstanzen der Formel (I) reduziert und gegebenenfalls mit anorganischen oder organischen Säuren in Salzform überführt.

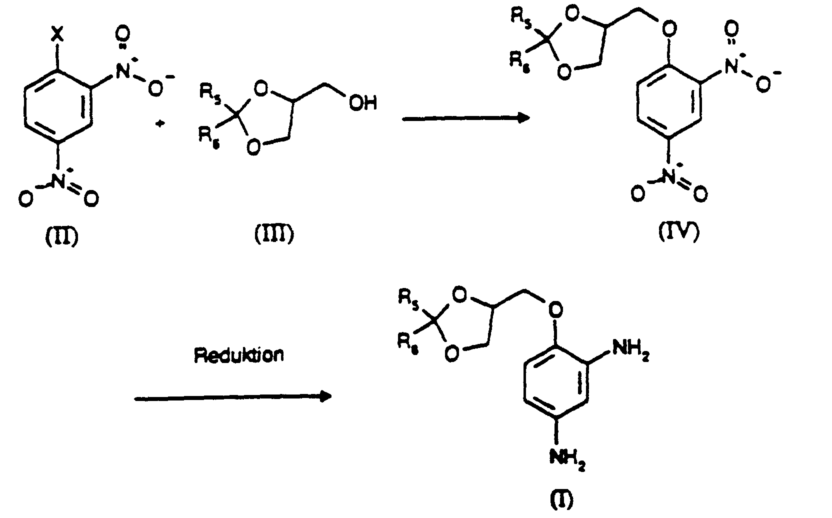

3. Verfahren zur Herstellung der Verbindungen der allgemeinen Formel (1), sowie ihre Salze mit anorganischen und organischen Säuren, worin R¹, R², R³, R⁴, R⁵ und R⁶ die bereits genannte Bedeutung besitzen, dadurch gekennzeichnet, daß man eine Verbindung der allgemeinen Formel (Va) oder (Vb), 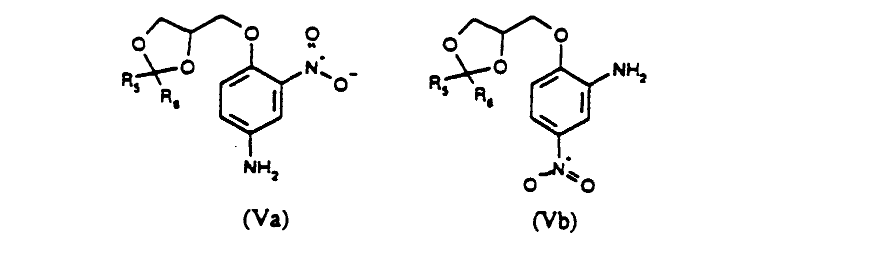 in einem inerten Lösungsmittel mit Chlorameisensäure-2-chlorethylester oder Chlorameisensäure-3-chlorpropylester zu den Verbindungen der allgemeinen Formel (VIa) oder (VIb) umsetzt, worin R⁷ = CH₂CH₂Cl oder CH₂CH₂CH₂Cl ist, 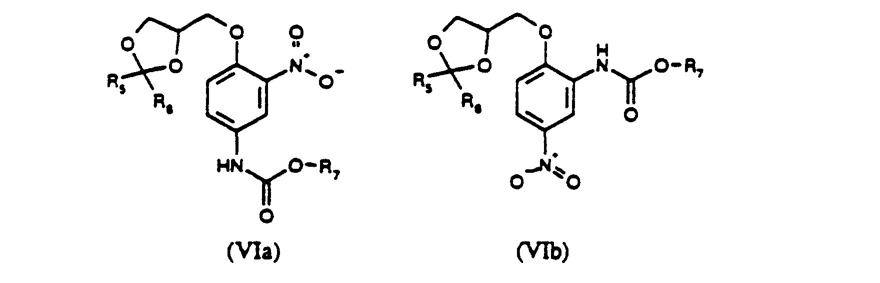 die in einem Lösungsmittel mit Basen zu den Verbindungen der allgemeinen Formel (VIIa) oder (VIIb), worin R⁸ = CH₂CH₂OH oder CH₂CH₂CH₂OH ist, umgesetzt werden, die mit bekannten 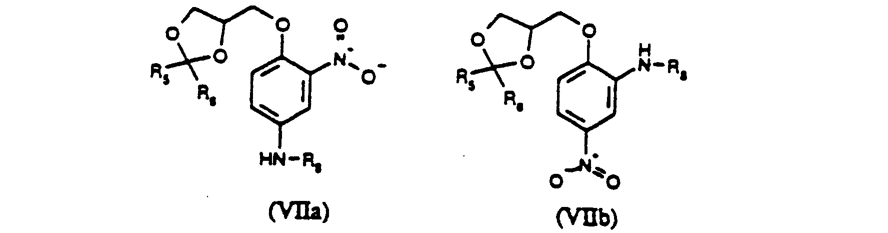 Alkylierungsmitteln oder Oxalkylierungsmitteln zu den Verbindungen der allgemeinen Formel (VIIIa) oder (VIIIb), wobei R² bis R⁵ die bereits angegebenen Bedeutungen haben, umgesetzt werden, die nach Reduktion und gegebenenfalls nach weiterer Alkylierung oder Oxalkylierung die Verbindungen der allgemeinen Formel (I) ergeben 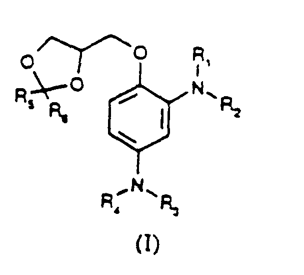 und diese gegebenenfalls mit einer anorganischen oder organischen Säure in ihr Salz überführt.

4. Haarfärbemittel auf der Basis der Verbindungen der allgemeinen Formel (I) in Form einer Creme, Lösung, Schaum oder Gels, die mindestens eine Verbindung der allgemeinen Formel (I) allein oder mit mindestens einem Entwickler enthalten.

5. Haarfärbemittel auf der Basis der Verbindungen der allgemeinen Formel (I) in Form einer Creme, Lösung, Schaum oder Gels, die mindestens eine Verbindung der allgemeinen Formel (I) allein oder mit mindestens einem Entwickler enthalten, bei dem die Oxidationsfärbung durch Zugabe eines Oxidationsmittels bewirkt wird.

6. Haarfärbemittel auf der Basis der Verbindungen der allgemeinen Formel (I) in Form einer Creme, Lösung, Schaum oder Gels, die mindestens eine Verbindung der allgemeinen Formel (I) allein oder mit mindestens einem Entwickler enthalten, bei dem die Oxidationsfärbung durch Zugabe eines Oxidationsmittels und eines Oxidationskatalysators bewirkt wird.

7. Haarfärbemittel auf der Basis der Verbindungen der allgemeinen Formel (I) in Form einer Creme, Lösung, Schaum oder Gels, die mindestens eine Verbindung der allgemeinen Formel (I) enthalten, in Abwesenheit eines Entwicklers.

8. Haarfärbemittel auf der Basis der Verbindungen der allgemeinen Formel (I) in Form einer Creme, Lösung, Schaum oder Gels, die mindestens eine Verbindung der allgemeinen Formel (I) allein oder mindestens einem Entwickler enthalten, und einen Oxydationskatalysator für die Bildung einer Oxidationsfärbung durch Luftsauerstoff aufweisen, wobei der Oxidationskatalysator aus einem Übergangsmetallsalz oder Übergangsmetallkomplex, insbesondere Kupfer(II)-chlorid, -sulfat, -acetat, allein oder als Addukt mit Ammoniak, Ethylendiamin, Phenanthrolin, Triphenylphosphin, 1,2-Diphenylphosphinoethan, 1,3-Diphenylphosphinopropan oder Aminosäuren einzeln oder im Gemisch besteht.

9. Haarfärbemittel, welche durch Luftoxidation eine Haaroxidationsfärbung erzeugen, in Form einer Creme, Lösung, Schaum oder Gels, die mindestens einen Kuppler der allgemeinen Formel (I) und mindestens einen Entwickler enthalten und als Oxidationskatalysator für Luftoxidation Kupfer(II)-1,2-diaminoethan-chlorid, Kupfer(II)-1,10-phenanthrolin-chlorid, Kupfer(II)-tetramminsulfat, Kupfer(II)-glycinat einzeln oder im Gemisch enthalten.

10. Haarfärbemittel nach Anspruch 9, in dem der Gehalt an Katalysator von 0.05 bis 0.5 Gewichtsprozent bezogen auf das Gewicht des Haarfärbemittels beträgt.

## Claims

1. Compounds corresponding to general formula (I): 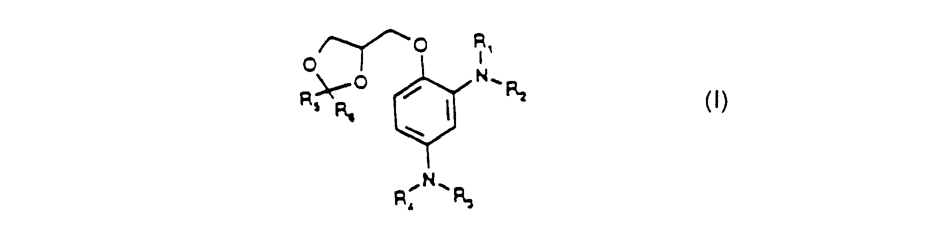 in which R¹, R², R³ and R⁴ independently of one another may represent a hydrogen atom, a (C₁₋₄) alkyl group, a hydroxy (C₂₋₃) alkyl group, an alkoxy (C₂₋₃) alkyl group, an amino (C₂₋₃) alkyl group or a 2,3-dihydroxypropyl group and R⁵ and R⁶ independently of one another may represent hydrogen or a (C₁₋₄) alkyl group,
and salts thereof with inorganic and organic acids.

2. A process for the production of compounds corresponding to general formula (I), characterized in that 2,4-dinitrohalobenzenes corresponding to formula (II), in which X = F, Cl, Br or iodine, are reacted with 4-hydroxymethyl-1,3-dioxolanes corresponding to formula (III), where R⁵ and R⁶ are as already defined, in an alkaline reaction medium to form 4-(2,4-dinitrophenoxymethyl)-1,3-dioxolanes corresponding to formula (IV) which are then reduced to form the secondary intermediates of formula (I) according to the invention and optionally converted into salts with inorganic or organic acids:

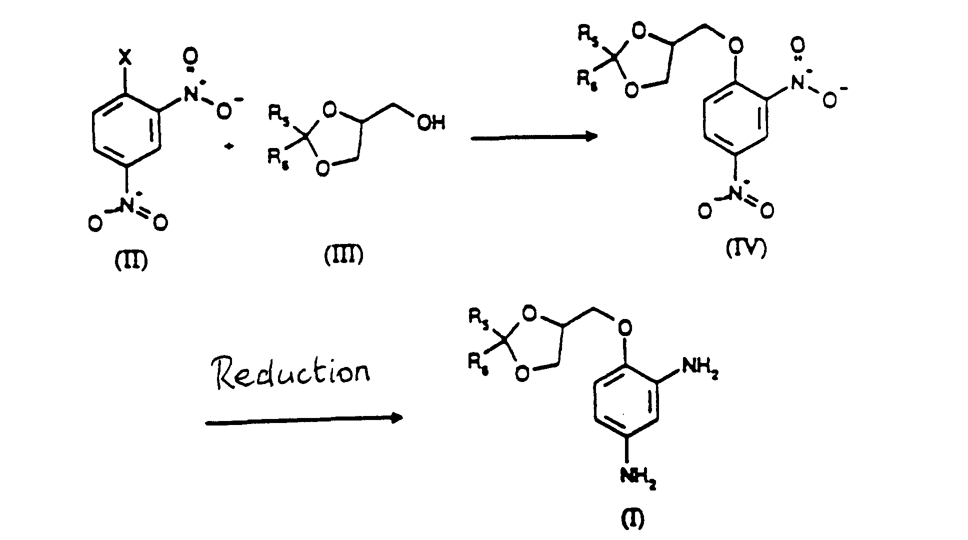

3. A process for the production of compounds corresponding to general formula (I), where R¹, R², R³, R⁴, R⁵ and R⁶ are as already defined, and salts thereof with inorganic or organic acids, characterized in that a compound corresponding to general formula (Va) or Vb): 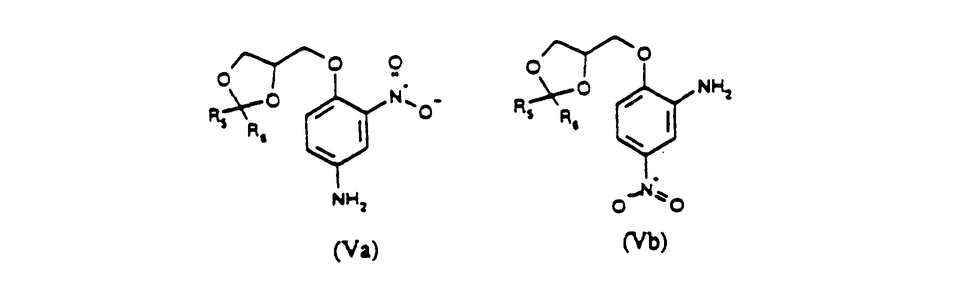 is reacted with chloroformic acid-2-chloroethyl ester or with chloroformic acid-3-chloropropyl ester in an inert solvent to form a compound corresponding to general formula (VIa) or VIb): 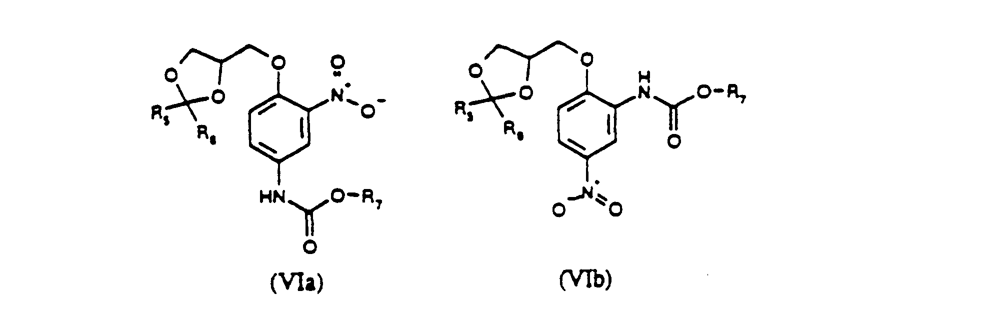 where R7 =CH₂CH₂Cl or CH₂CH₂CH₂Cl,
the compound (VIa) or (VIb) thus obtained is reacted with bases in a solvent to form a compound corresponding to general formula (VIIa) or (VIIb): 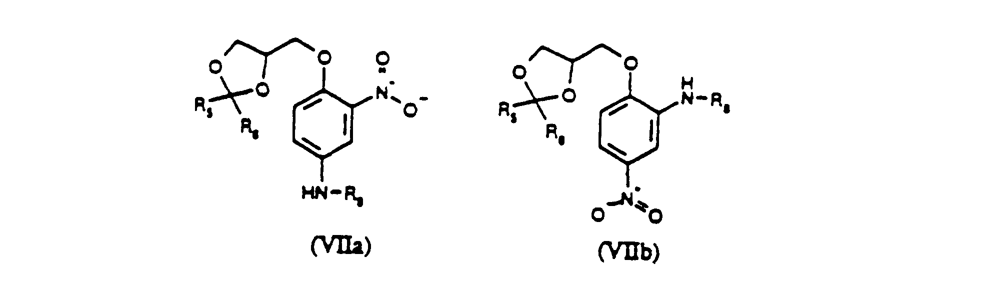 where R⁸ = CH₂CH₂OH or CH₂CH₂CH₂OH,
the compound (VIIa) or (Vllb) obtained is reacted with an alkylating agent or alkoxylating agent to form a compound corresponding to general formula (VIIIa) or (VIIIb), where R² to R⁵ are as already defined, the compound (VIIIa) or (VIIIb) obtained is reduced and, optionally after further alkylation or alkoxylation, gives the compounds corresponding to general formula (I): 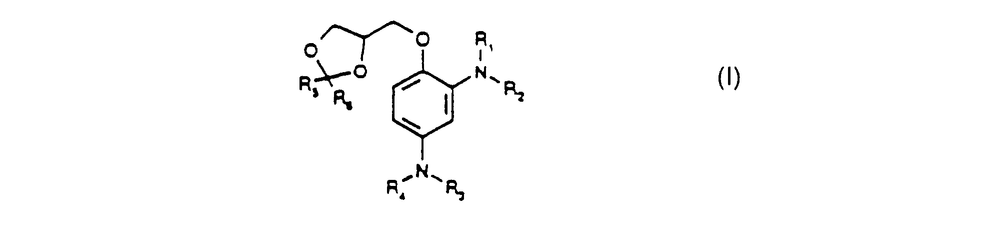 which are optionally converted into salts with an inorganic or organic acid.

4. A hair colorant based on the compounds corresponding to general formula (I) in the form of a creme, solution, foam or gel which contain at least one compound corresponding to general formula (I) either on its own or in combination with at least one primary intermediate.

5. Hair colorants based on the compounds corresponding to general formula (I) in the form of a creme, solution, foam or gel which contain at least one compound corresponding to general formula (I) on its own or in combination with at least one primary intermediate where oxidation coloring is effected by addition of an oxidizing agent.

6. Hair colorants based on the compounds corresponding to general formula (I) in the form of a creme, solution, foam or gel which contain at least one compound corresponding to general formula (I) on its own or in combination with at least one primary intermediate where oxidation coloring is effected by addition of an oxidizing agent and an oxidation catalyst.

7. Hair colorants based on the compounds corresponding to general formula (I) in the form of a creme, solution, foam or gel which contain at least one compound corresponding to general formula (I) in the absence of a primary intermediate.

8. Hair colorants based on the compounds corresponding to general formula (I) in the form of a creme, solution, foam or gel which contain at least one compound corresponding to general formula (I) on its own or in combination with at least one primary intermediate and an oxidation catalyst for forming an oxidation color under the effect of atmospheric oxygen, the oxidation catalyst consisting of a transition metal salt or transition metal complex, more particularly copper(II) chloride, sulfate or acetate as such or as an adduct with ammonia, ethylenediamine, phenanthroline, triphenyl phosphine, 1,2-diphenyl phosphinoethane, 1,3-diphenyl phosphinopropane or amino acids either individually or in the form of a mixture.

9. Hair colorants in the form of a creme, solution, foam or gel which produce an oxidation hair color by oxidation with air and which contain at least one secondary intermediate corresponding to general formula (I) and at least one primary intermediate and, as oxidation catalyst for oxidation with air, copper(II)-1,2-diaminoethane chloride, copper(II)-1,10-phenanthroline chloride, copper(II)-tetrammine sulfate, copper(II)-glycinate either individually or in the form of a mixture.

10. Hair colorants as claimed in claim 9, in which the catalyst content is between 0.05 and 0.5% by weight, based on the weight of the hair colorant.

## Revendications

1. Composés de formule générale (I) ainsi que leurs sels avec un acide minéral et organique, 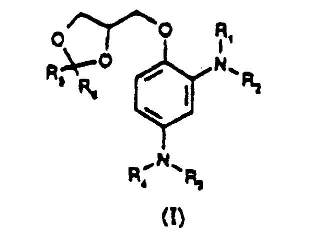 dans laquelle R¹, R², R³ et R⁴ sont indépendamment les uns des autres, un atome d'hydrogène, un groupe alkyle en C₁-C₄, un groupe hydroxyalkyle en C₂-C₃, un groupe alkoxyalkyle en C₂-C₃, un groupe aminoalkyle en C₂-C₃, un groupe 2,3-dihydroxypropyle, et R⁵ ainsi que R⁶, indépendamment l'un de l'autre, peuvent représenter de l'hydrogène ou un radical alkyle en C₁-C₄.

2. Procédé de préparation de composés de formule générale (I),
caractérisé en ce qu'
on fait réagir des 2,4-dinitro-halogénobenzènes de formule (II) dans laquelle X = F, Cl, Br ou de l'iode, avec des 4-hydroxyméthyl-1,3-dioxolanes de formule (III) dans laquelle R⁵ et R⁶ possèdent la signification déjà citée, dans un milieu réactionnel alcalin, pour les convertir en 4-(2,4-dinitrophénoxyméthyl)-1,3-dioxolanes de formule (IV), et on réduit ceux-ci en les substances de couplage de formule (I) conformes à l'invention, et le cas échéant, sous forme de sel avec des acides minéraux ou organiques.

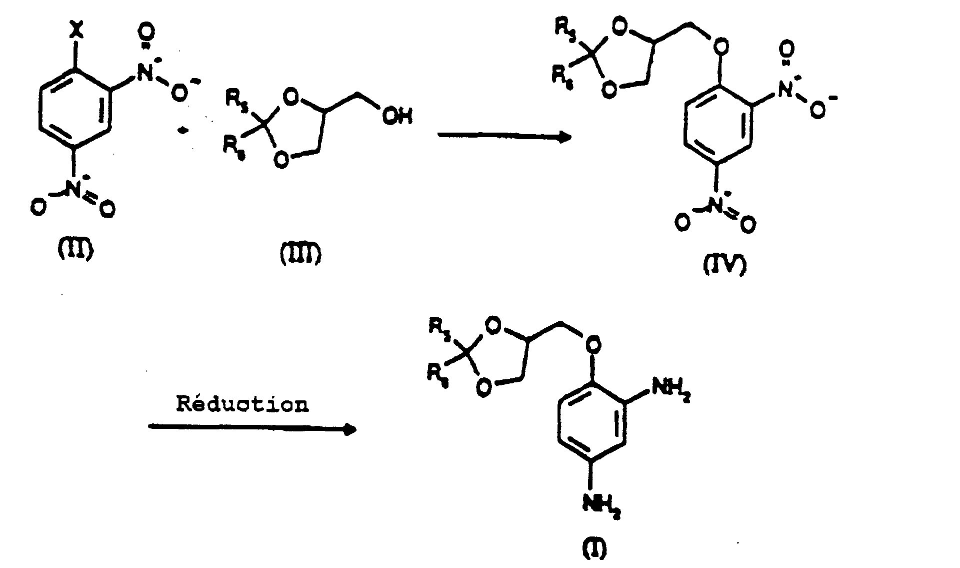

3. Procédé de préparation des composés de formule générale (I) ainsi que de leurs sels avec des acides minéraux et organiques, dans lesquels R¹, R², R³, R⁴, R⁵ et R⁶ possèdent la signification déjà citée,
caractérisé en ce qu'
on fait réagir un composé de formule générale (Va) ou (Vb), 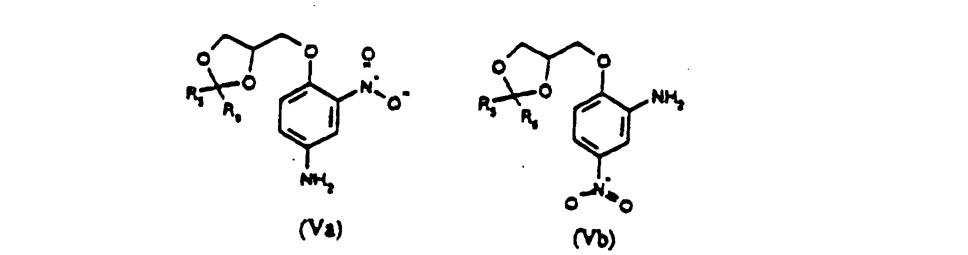 dans un solvant inerte avec l'ester 2-chloroéthylique d'acide chloroformique ou avec l'ester 3-chloropropylique d'acide chloroformique, pour obtenir des composés de formule générale (Via) ou (VIb) dans lesquelles R⁷ est = CH₂CH₂Cl ou CH₂CH₂CH₂Cl, 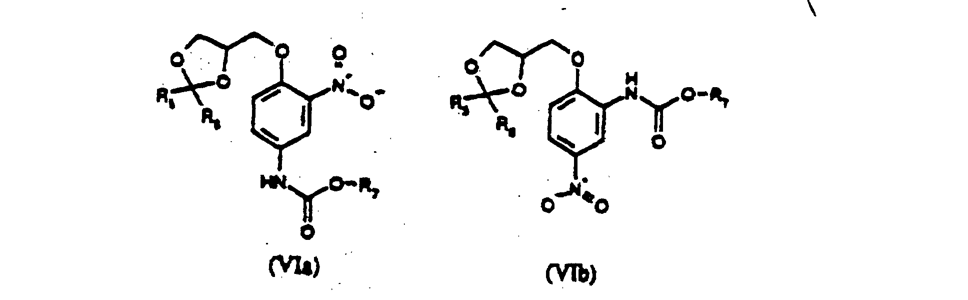 qui sont convertis dans un solvant avec des bases, en des composés de formule générale (VIIa) ou (VIIb) dans lesquelles R⁸ est = CH₂CH₂OH ou CH₂CH₂CH₂OH, 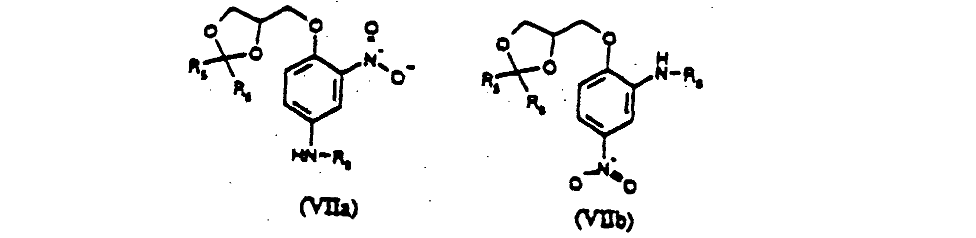 qui sont transformés avec des agents d'alkylation ou des agents d'oxalkylation connus, en des composés de formule générale (VIIIa) ou (VIIIb) dans lesquelles R² à R⁵ ont les significations déjà indiquées, qui après réduction ou éventuellement après une alkylation ou une oxalkylation supplémentaire fournissent les composés de formule générale (I), 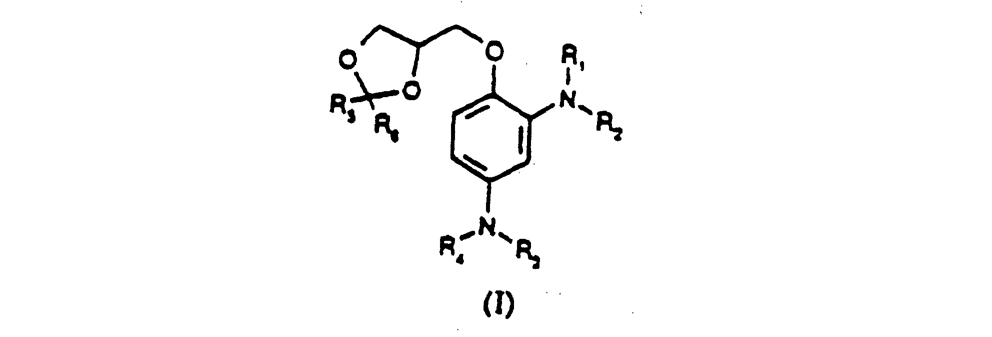 et on convertit ceux-ci éventuellement en leur sels avec des acides minéraux ou organiques.

4. Teintures pour cheveux à base de composés de formule générale (I) sous forme d'une crème, d'une solution, d'une mousse ou de gels, qui renferment au moins un composé de formule générale (I) seul ou avec au moins un agent de développement.

5. Teintures pour cheveux à base des composés de formule générale I sous forme d'une crème, d'une solution, d'une mousse ou de gels qui renferment au moins un composé de formule générale (I) seul ou avec au moins un agent de développement grâce auquel la coloration par oxydation est occasionnée par addition d'un agent par oxydation.

6. Teintures pour cheveux à base de composés de formule générale (I) sous forme d'une crème, d'une solution, d'une mousse, ou de gels qui renferment au moins un agent de développement, grâce auquel la coloration par oxydation est occasionnée par addition d'un agent d'oxydation et d'un catalyseur d'oxydation.

7. Teintures pour cheveux à base de composés de formule générale (I) sous forme d'une crème, d'une solution, d'une mousse ou de gels, qui renferment au moins un composé de formule générale (I) en l'absence d'un agent de développement.

8. Teintures pour cheveux à base de composés de formule générale (I) sous forme d'une crème, d'une solution, d'une mousse ou de gels, qui renferment au moins un composé de formule générale (I) seul ou au moins un agent de développement, et qui possèdent un catalyseur d'oxydation pour la formation d'une coloration par oxydation par l'oxygène atmosphérique, dans lesquelles le catalyseur d'oxydation consiste en un sel de métal de transition ou en un complexe de métal de transition, en particulier le chlorure, le sulfate, l'acétate de cuivre(II) seul ou sous forme de produit d'addition avec l'ammoniac, l'éthylènediamine, la phénanthroline, la triphénylphosphine, le 1,2-diphénylphosphinoéthane, le 1,3-diphénylphosphinopropane, ou des aminoacides isolément ou en mélange.

9. Teintures pour cheveux produisant une coloration par oxydation à l'air, sous forme d'une crème, d'une solution, d'une mousse ou de gels, qui renferment au moins un coupleur de formule générale (I) et au moins un agent de développement, et qui contiennent comme catalyseur d'oxydation pour l'oxydation à l'air, le chlorure de cuivre(II)-1,2-diaminoéthane, le chlorure de cuivre(II)-1,10-phénanthroline, le sulfate de cuivre(II)-tétraamimine, le glycinate de cuivre(II), isolément ou en mélange.

10. Teintures pour cheveux selon la revendication 9,
dans lesquelles
la teneur en catalyseur s'élève de 0,05 à 0,5 % en poids, rapporté au poids de la teinture pour cheveux.
